# EUROPEAN PATENT APPLICATION

(11) **EP 1 118 339 A1**
(43) Date of publication of application: **25.07.2001**
(21) Application number: 00100257.5
(22) Date of filing: 18.01.2000
(51) Int. Cl.: A61L 15/36, A61F 13/15, B32B 9/00

(54) **Breathable absorbent articles comprising spores which exhibit antagonistic properties against pathogens**

(71) Applicant: THE PROCTER & GAMBLE COMPANY, Cincinnati, Ohio 45202 (US)
(72) Inventor: Di Cintio, Achille, 65126 Pescara (IT); Carlucci, Giovanni, 66100 Chieti (IT); Pesce, Antonella, 65120 Pescara (IT)
(74) Representative: Kremer, Véronique Marie Joséphine

(57) **Abstract**

The present invention relates to breathable absorbent articles, such as sanitary napkins, pantiliners, nursing pads and baby diapers having a breathable backsheet, and comprising spores which have the ability to germinate into microorganisms which exhibit antagonistic properties against undesirable strains of microorganisms. A storage stable breathable absorbent article is provided that can be worn for long periods of time without occurrence of undesirable odors while providing high protection level.

## Description

### Field of the Invention

The present invention relates to breathable absorbent articles, particularly sanitary napkins and panty liners, which comprise spores which in their living form exhibit antagonistic properties against undesirable strains of microorganisms.

### Background of the Invention

Whilst the primary focus of absorbent articles, in particular sanitary napkins remains the ability of these articles to absorb and retain fluids, another important area of development in this field is the control of odorous compounds contained within the absorbed articles during their use. Malodorous compounds typically present in absorbent articles originate from a number of sources. Firstly, the actual components of the bodily fluid discharge such as urine, perspiration, menstrual fluids, menstrual blood and vaginal discharges may themselves contain malodorous compounds. Secondly, malodorous compounds are also generated as a result of the degradation of the components of the bodily fluid discharge contained within the absorbent articles. Consequently, there are a wide range of compounds which may be present at some time during the use of an absorbent article which have an associated malodour. These compounds include volatile fatty acids, ammonia, amines, sulphur containing compounds, alcohol, ketones and aldehydes and numerous derivatives thereof.

The presence and detection of malodorous compounds from absorbent articles during their use, particularly those associated with menstruation may cause the wearer of these articles embarrassment. Thus, the prevention of the detection of malodour from such articles is highly desirable.

As a result there are numerous disclosures in the art which describe various agents which provide odour control for use in absorbent articles. These odour control agents typically function by physical absorption of the odorous compounds or by chemical interaction with the odorous compounds or precursors of odorous compounds or by masking the odour.

Most of the focus in the prior art is found on the odour absorption technology. Examples of these types of compounds include activated carbons, clays, zeolites, silicates, starches, cyclodextrine, ion exchange resins and various mixture thereof as for example described in EP-A-348 978, EP-A-510 619, WO 91/12029, WO 91/11977, W089/02698, and/or WO 91/12030. All of these types of odour absorbing agents are believed to control odour by mechanisms whereby the malodorous compounds and their precursors are physically absorbed by the agents and thus such agents hinder the exit of the odour from articles like absorbent articles.

However, such odour-absorbing agents are not completely effective as the formation of the odour itself is not prevented and thus odour detection is not completely avoided. In presence of such odour-absorbing agents the pathogenic bacteria are still able to grow, and the use of bacteria inhibiting agents, which are often selective, can create risks, for instance, in the form of allergenic properties or negative ecological consequences when handling waste. Furthermore, the use of bacteria inhibiting agents involves the risk that resistant strains will occur.

WO 92/13577 and WO 97/02846 disclose the use of lactic acid producing bacteria in absorbent articles to reduce the growth of undesirable pathogens and hence reduce the formation of malodorous compounds.

However, the problem encountered with lactic-acid producing bacteria-containing articles is the stability of the articles both during storage and use. None of these two prior art documents discloses anything about providing absorbent articles which provide effective odour control properties in use condition, which articles can be stored for a long time and still contain a sufficient amount of active and transferable bacteria. It is absolutely necessary that consumer products such as absorbent articles can be stored for a long time and under non-ideal conditions without risking that the quality of the articles is impaired.

Hence, there still exists a need to provide alternative odour control agents or systems for effective utilization in absorbent articles.

It is an object of the present invention to provide absorbent articles, which provide effective odour control over a broad spectrum of malodorous compounds while being stable over prolonged time of storage, i.e., without risking that the quality of the article is impaired before actual use (typically before it comes into contact with bodily fluids).

It is a further object of the present invention to provide absorbent articles, which deliver sustained odour control over prolonged use time of the article, i.e., a long lasting odour control by preventing the formation of odour over prolonged wearing time of the article.

Finally, it is an object of the present invention to provide absorbent articles having the benefits mentioned herein before while meeting the requirements of ease of manufacturing, time effectiveness and cost effectiveness.

These objects have been achieved in accordance with the present invention by providing a breathable absorbent article, particularly by the provision of a breathable backsheet, which article comprises spores which are able to germinate under favorable conditions (typically in use, e.g., when the article is applied to the external surface of a human or animal) in microorganisms (living form of the corresponding spores) which exhibit antagonistic properties against undesirable strains of microorganisms, typically present or arisen in the article or on the surface (e.g, skin or urogenital zone of a human) the article is applied to.

The living form of the spores have an activity such as to restrain the growth of undesirable strains of microorganisms or establishing of new undesirable species of microorganisms. Even some killing of microorganisms of undesirable species may occur. Indeed, the living form of the spores used in the present invention, preferably the spores *of L. sporogenes,* have the ability to inhibit pathogenic microorganisms by competing for substrate. The antagonistic properties of the living form of the spores used herein is partly denoted their ability of producing different so called antimetabolites, such as lactic acid, lactoperoxidases, bacteriocins and carbon dioxides.

More particularly, it has surprisingly been found that the use, in a breathable absorbent article, of the spores of microorganisms which exhibit antagonistic properties against undesirable pathogens (preferably L. sporogenes spores), provides an effective odour controlling article which can be stored for long time periods before its actual use, without risking that the odour control ability of the article in use is impaired. The use of these spores able storage stability upon prolonged periods of time up to the time the article is used, i.e., is applied on the external surface of a human or animal body where it typically comes into contact with bodily fluids, and undergoes environmental changes that will create favourable environmental conditions for the germination of the spores. In use conditions, for example when the article is applied onto the skin or in the urogenital zone, the availability of nutriments like menses, vaginal discharge, urine, and the like, will allow transformation/germination of the spores (dormant form) in the microorganisms (living form).

It is believed that the breathable environment contributes to the effective odour control benefits associated with the articles according to the present invention. Indeed the breathability of the article, which reduces the hot, humid and occlusive environment between the skin of the wearer and the surface of the absorbent article, reduces the tendency of the wearer to perspire. This results in a twofold benefit. Firstly, the amount of associated perspiration related odour will be reduced. Thus, the breathability of the article actually reduces the amount of odour generated within the absorbent article. Secondly, reduced perspiration will maintain the spores in their dormant form up to contact with bodily fluid discharge (menstruation, vaginal discharge and/or urine), and thus maintain the whole odour control capacity up to the time bodily fluids contact the absorbent article.

Advantageously, the articles according to the present invention retain their whole odour control capacity up to the time they come in contact with bodily fluids. Thus the present invention also provides articles with effective odour control capacity, while using reduced total amount of active material, i.e., odour control material.

A further advantage associated with the absorbent articles of the present invention like pantiliners or pads, is a better feeling and more acceptable dryness and cleanness level in use. Indeed, it has further been found that in the living form of the spores herein allow gelification of the bodily fluid discharge of a person wearing such an absorbent article, thereby facilitating fluid transport into the article. In a preferred embodiment the spores are placed in the core of the absorbent article, thereby changing the physical properties of the bodily fluids. Such gelification will have the benefits of reducing the rewetting of the topsheet and the soiling through as might otherwise result from squeezing the article as a consequence of legs movement of a person wearing the article.

The breathable backsheet of the article further contributes to the dryness/cleanness benefit associated with the presence of the spores herein. The breathability of the article allows for the evaporation of fluid from the article, and also as indicated above a reduction in the amount of perspiration generated by the wearer of the product and thus a reduction in hot and sweaty feelings often associated with the presence of topsheets designed to retain a clean and dry surface. The article therefore needs to retain less fluid and can do so more effectively. Furthermore the use of the breathable backsheet further contributes to a clean and dry facing surface (topsheet), such that the surface feels dry to the touch and the skin of the wearer of the article does not feel wet or moist and such that the wearer experiences minimal discomfort during wearing.

### Summary of the Invention

The present invention relates to an absorbent article having a breathable backsheet and further comprising spores which have the ability to germinate into micro-organisms which exhibit antagonistic properties against undesirable strains of micro-organisms, typically present or arisen in the article or on the surface the article is applied to.

The present invention also encompasses the use of spores which have the ability to germinate into micro-organisms which exhibit antagonistic properties against undesirable strains of micro-organisms, in a breathable absorbent article, comprising a liquid permeable topsheet, an absorbent core and a breathable backsheet, for reducing leakage/wet through.

In its broadest embodiment the present invention also encompasses the use of spores which have the ability to germinate into micro-organisms which exhibit antagonistic properties against undesirable strains of micro-organisms, in an absorbent article, preferably a breathable absorbent article, comprising a wearing facing surface and a garment facing surface for improved dryness of the wearer facing surface.

### Detailed Description of the Invention

The present invention relates to breathable absorbent articles such as sanitary napkins, panty liners, incontinence devices, nursing pads/breast pads and baby diapers, interlabial pads. Typically such products comprise a liquid pervious topsheet, a backsheet and an absorbent core intermediate the topsheet and the backsheet. According to the present invention the breathability of the absorbent article is provided by the presence of a breathable backsheet which thereby allows the circulation of water vapor and preferably both water vapor and air through it. According to the present invention the absorbent article comprises spores which have the ability to germinate into micro-organisms which exhibit antagonistic properties against undesirable strains of micro-organisms, preferably spores from *L sporogenes.* It has now been found that the combination of the breathability of the absorbent article and the spores herein results in storage stable articles delivering outstanding odour control properties with an effective level of protection (i.e., reduced leakage/wet through).

By "bodily fluids" it is meant herein any fluid produced by human body including for instance urine, menstrual fluids, faeces, vaginal secretions and the like.

### The spores

As an essential element the absorbent article of the present invention comprises a spore which germinates in a microorganism which exhibits antagonistic properties against undesirable strains of microorganisms, typically present or arisen in the article or on the surface the article is applied to (e.g., skin or urogenital zone of a wearer).

The spores also called endospores to be used herein are the dormant form of spore-forming lactic acid producing micro-organisms (living form) (also called herein after spore-forming antagonistic micro-organisms). These spores/endospores are heat-resistant, dehydrated resting cells that are formed intracellularly and contain a genome and all essential metabolic machinery. The spores are encased in a complex protective spore coat. Preferred micro-organisms able to form such spores for use herein are the species *Lactobacillus Sporogenese* and those belonging to the genus Sporolactobacillus, more particularly the species *Sporolactobacillus inulinus*. Highly preferred herein is the species *L.sporogenes.*

*L. sporogenes* was first isolated from green malt and described in 1933 by L.M. Horowitz-Wlassowa and N.W. Nowotelnow. It was submitted as L. *sporogenes* in the fifth edition (1939) of 'Bergey's manual of Determinative Bacteriology' as well as mentioned in recognized scientific publication, Korean J. Appl. Microb. & Bioengin. (1985) 13:185-190, J. Pharmaceut. Soc. Korea (1977) vol XXIII, 1-Feb, 473-474. *L. sporogenes* was transferred to *Bacillus coagulans* in the seventh edition of 'Bergey's manual of Determinative Bacteriology' due to simplification in cataloguing. However in honour of the original discoverers the name *L. sporogenes* is used widely. Reference is also made to the taxonomical classification of *Sporolactobacillus* in L'integratore Nutrizionale 2 (1) 1999, Stabilita' di integratori con Sporolactobacillus, classificazione tassonomica by L. Marossi and all.

According to the Eighth Edition of Bergey's Manual of Determinative Bacteriology, "various spore-bearing rods which produce lactic acid, are facultative or aerobic and catalase positive, have generally and correctly been assigned to the genus *Bacillus'.* The characteristics of L. *sporogenes* as cited in 'Bergey's Manual of Determinative Bacteriology' (seventh edition) and other sources are "gram positive spore-forming rods 0.9 by 3.0 to 5.0 micron size, aerobic to microaerophilic, producing L (+) lactic acid homofermentatively". *L. Sporogenes* also releases other metabolites like carbon dioxide, diacetyl, bacteriocins, lacto-peroxidase.

Since *L. Sporogenes* exhibits characteristics typical of both genera *Lactobacillus* and *Bacillus,* its taxonomic position between the families *Lactobacillaceae* and *Bacillaceae* has often been discussed. This along with the fact that there is no universally accepted official classification leaves room for controversy in the nomenclature. More information about *L.sporogenes* is available from the commercial brochure 69/107, incorporated herein by reference, of Sochim International s.p.a. Milano, a supplier of the spore form of *L. Sporogenes.*

Some authors refer to *L.sporogenes* as *Bacillus coagulans. Bacillus coagulans* Hammer deposited as *Lactobacillus sporogenes* by Kabushiki Kaisha Naruse Fermentation Research Laboratory is commercially available under ATCC number 31284 (Internet information source : http://www.atcc.org/). ATCC stands for American Type Culture Collection.

The spores for use herein are typically activated due to the presence of substrate, temperature increase and pH change. The optimum growth temperature range for the spores of *L. sporogenes* is between 30°C and 50°C and the optimum pH range is from 5.0 to 6.5. Thus perspiration (pH 6) and fluid discharge like menstrual fluid discharge (pH 6.5), milk discharge (pH 6.4 ) and/or urine discharge (pH 6.4) will active the germination of the spores herein.

*L.sporogenes* spores are ellipsoidal bodies measuring 0.9 to 1.2 by 1.0 to 1.7 microns. These spores are commercially available in a white to greyish powder form. *L. sporogenes* spores powder is commercially available under the name LACTOSPORE® from SABINSA CORPORATION (Sochim international s.p.a, Milano). 1 gram of Lactospore® corresponds to about 15^{*}10⁹ of spores.

It is necessary for the spores to be added in amounts which will achieve the desired effect under favorable conditions. It is necessary for the spores to be added in amounts which will achieve the desired effect under favorable conditions. Typically, such spores are present in the article herein at a level per article of more than 10², preferably more than 10⁴, more preferably from 10⁵ to 10¹² and most preferably from 10⁶ to 10¹⁰.

This typically will correspond to a number of spore-forming lactic acid-producing microorganisms per article which exceeds 10² cfu, preferably exceeds 10⁴ cfu, more preferably from 10⁵ cfu to 10¹² cfu and most preferably from 10⁶ to 10¹⁰ cfu.

Malodour is generated among other by inter or extra cellular metabolic activity which satisfy the bacteria needs for energy and proliferation. The metabolic activity aiming the production of vital oxygen, carbon and ammonium sources, leads to degraded odorous compounds as by products which are among low molecular weight of fatty acids, amines, mercaptan, indoles, ammonium, sulfides and the like. Bacteria that cause unpleasant smells may belong to, for example, the family Enterobacteriaceae, e.g., Proteus mirabilis, Proteus vulgaris, Escheriachia coli and Klebsiella.

The particularities of the spores used herein is based on the microbiological antagonism of their living form. This implies that such antagonistic microorganisms inhibit other microorganisms by competing for substrates, forming metabolites like L (+) form of lactic acid, enzymes like lacto-peroxidases, toxins, carbon dioxide, peroxides or antibiotics, so-called bacteriocines. The spores used herein have the ability to sustain the growth and reduce the patogenicity of many pathogens like ones mentioned herein before and especially Proteus, Pseudomonas, Echerichia, Klebsiella, Enterococcus, Staphylococcus, Streptococcus and Candida.

It is speculated that he production of lactic acid lowers the pH to 4-5, thereby inhibiting the growth of putrefactive organisms like E.coli, which require a higher optimum pH (typically 6 to 7) for favourable growth conditions. Furthermore undissociated lactic acid has the tendency to penetrate the membrane of pathogens, lowering their intracellular pH and/or interfering with their metabolic processes such as oxidative phosphorylation, thereby inhibiting the growth of such pathogens

Other metabolites further contribute to inhibit the growth of pathogens. For example carbon dioxide is believed to reduce membrane permeability. Hydrogen peroxide / Lactoperoxidase are believed to oxidise basic proteins and to destroy the "enzymes factories" (ribosomes) of pathogens. Bacteriocins are proteins or protein complexes with bactericidal activity. Indeed, bacteriocins have the ability to link to particular receptors on the cell wall of microorganisms, thereby affecting the functionality of the cell wall/membranes. Bacteriocins are also able to affect DNA-synthesis and protein synthesis.

The particularity of the spores of the antagonistic microorganisms herein is that they give antagonistic microorganisms that may be naturally occurring microorganisms that are non-toxic and do not have any negative biological effect on humans.

One advantage afforded by the use of antagonistic microorganisms is that there is avoided an undesired selection pressure on the micro environment, such as favoring potential desease-promoting microorganisms and therewith the risk of developing pathogenic strains that are resistant to antibiotics and chemopharmaceutical preparations. Since the antimicrobial system is based on a natural, biological process, there is less risk of environmental ecological and toxic disturbances.

Advantageously it has been found that the present breathable absorbent articles can be stored for long time before actual use and still contain their whole odor control capacity up to the time the articles are used. Indeed the antogonistic microorganisms used according to the present invention like *L. sporogenes* are incorporated in the article in their spore form. Indeed *L. sporogenes cells* (in spore form) are protected from destruction by environmental factors by the naturally-present microencapsulation system, the spore coat. These spores are activated by environmental changes like pH and temperature changes and availability of substrate. For instance in use when the article is contacted with the skin or the urogenital zone of a wearer, the temperature increases (from room temperature to about 30°C-35°C) and substrates like bodily fluid discharge are provided, such conditions will activate the spores germination. The spore coat imbibe water, swell and the increased water content will cause a rise in the metabolic rate of the sporulated bacili. Outgrowths will begin to protrude from the spore-coats. The outgrown cells germinate and transform into viable vegetative cell (also called 'living form' herein). The living form begin to proliferate multiplying rapidly. These living forms continue their metabolic activities producing lactic acid and other metabolites which render the environmental non-conducive for the growth of harmful pathogenic micro-organisms. The germination process will be influenced by the body discharge in the article, leading to a somehow controlled germination process on demand. In other words all the spores will not germinate with the same kinetic but the germination will be in relation to the body fluid discharge on the article. This will contribute to prolonging the effective beneficial microbial activity.

Advantageously by using the spores herein in the absorbent articles, the genetic identity, the lactic acid producing ability and viability of the microorganisms are maintained upon prolonged periods of use. Indeed once in their living form these microorganisms have the ability to transform again in spores and accordingly re-germinate in time sequence in line with further bodily fluid discharge. Advantageously the spores herein have the ability to germination and re-germination in line with the bodily fluid discharge, this mechanisms results in long lasting odour control benefits.

An important advantage of the spores used according to the present invention is that the spores can be stored at room temperature (20°-25°C) without loss of viability as opposed to non-spore forming lactic acid producing bacteria taken from the genera Lactobacillus or Lactococcus like Lactobacillus acidophilus, Lactococcus lactis and the like. In the case of such non-sporulated antagonistic species, attempts have been made to retain the viability of the vegetative cells during prolonged storage by freeze-drying. These cells have to be stored under adequate refrigeration and are susceptible to environmental damage.

Advantageously it has now been found that the spores as described herein have the advantage of changing the physical properties of bodily fluid. Indeed a gelification of the bodily fluid is obtained when the fluid comes into contact with the spores. This results in reduced leakage/wet through of the breathable absorbent articles.

Without to be bound by theory, it is speculated that the cause of this gelification is the denaturation of the proteins. Indeed the spores herein upon activation and germination in their living form/microorganism release lactic acid through glycogen fermentation. As proteins are sensitive to pH, the presence of lactic acid causes the soluble proteins contained into the bodily fluid to turn into insoluble form. This creates a sort of tri-dimensional net of molecules trapping globules, minerals, fats which results in the so called gelification of the bodily fluid.

In its broadest aspect the present invention is based on the finding that the use of spores as described herein in an absorbent article comprising a garment facing surface and a wearer facing surface provides improved dryness of the wearer facing surface. By improved dryness reference is made to the same absorbent article in absence of such spores. As used herein the tospheet provides said wearer facing surface and the backsheet provides the garment facing surface. Improved dryness and cleanness is particularly noticed on the wearer facing surface in those embodiments wherein the spores are located in the absorbent core.

### Optional agents

The articles according to the present invention may further comprise on top of the spores described herein before, other conventional agents or mixtures thereof.

### Absorbent gelling materials

As is well-known from recent commercial practice, absorbent gelling materials (sometimes referred to as "super-sorbers") are becoming broadly used in absorbent articles. AGM's are materials which have fluid-absorbing properties.

Such materials are highly preferred herein due to their dual function of absorbing and retaining fluids and odors.

Such materials form hydrogels on contact with water (e.g., with urine, blood, and the like). One highly preferred type of hydrogel-forming, absorbent gelling material is based on polyacids, especially polyacrylic acid. Hydrogel- forming polymeric materials of this type are those which, upon contact with fluids (i.e., liquids) such as water or body fluids, imbibe such fluids and thereby form hydrogels. These preferred absorbent gelling materials will generally comprise substantially water-insoluble, slightly cross-linked, partially neutralized, hydrogel-forming polymer materials prepared from polymerizable, unsaturated, acid-containing monomers. In such materials, the polymeric component formed from unsaturated, acid-containing monomers may comprise the entire gelling agent or may be grafted onto other types of polymer moieties such as starch or cellulose. Acrylic acid grafted starch materials are of this latter type. Thus, the preferred absorbent gelling materials include hydrolyzed acrylonitrile grafted starch, acrylic acid grafted starch, polyacrylates, maleic anhydride-based copolymers and combinations thereof. Especially preferred absorbent gelling materials are the polyacrylates and acrylic acid grafted starch.

Whatever the nature of the polymer components of the preferred absorbent gelling materials, such materials will in general be slightly cross-linked. Crosslinking serves to render these preferred hydrogel-forming absorbent materials substantially water-insoluble, and cross-linking also in part determines the gel volume and extractable polymer characteristics of the hydrogels formed therefrom. Suitable cross-linking agents are well known in the art and include, for example, (1) compounds having at least two polymerizable double bonds; (2) compounds having at least one polymerizable double bond and at least one functional group reactive with the acid-containing monomer material; (3) compounds having at least two functional groups reactive with the acid-containing monomer materials; and (4) polyvalent metal compounds which can from ionic cross-linkages. Cross-linking agents of the foregoing types are described in greater detail in Masuda et al; U.S. Patent 4,076,663; Issued February 28, 1978. Preferred cross-linking agents are the di- or polyesters of unsaturated mono-or polycarboxylic acids with polyols, the bisacrylamides and the di-or triallyl amines. Especially preferred cross-linking agents are N,N'-methylenebisacrylamide, trimethylol propane triacrylate and triallyl amine. The cross-linking agent will generally comprise from about 0.001 mole percent to 5 mole percent of the preferred materials. More preferably, the cross-linking agent will comprise from about 0.01 mole percent to 3 mole percent of the gelling materials used herein.

The preferred, slightly cross-linked, hydrogel-forming absorbent gelling materials will generally be employed in their partially neutralized form. For purposes described herein, such materials are considered partially neutralized when at least 25 mole percent, and preferably at least 50 mole percent of monomers used to form the polymer are acid group-containing monomers which have been neutralized with a salt-forming cation. Suitable salt-forming cations include alkali metal, ammonium, substituted ammonium and amines. This percentage of the total monomers utilized which are neutralized acid group-containing monomers is referred to as the "degree of neutralization". Typically, commercial absorbent gelling materials have a degree of neutralization somewhat less than 90%.

The preferred absorbent gelling materials used herein are those which have a relatively high capacity for imbibing fluids encountered in the absorbent articles; this capacity can be quantified by referencing the "gel volume" of said absorbent gelling materials. Gel volume can be defined in terms of the amount of synthetic urine absorbed by any given absorbent gelling agent buffer and is specified as grams of synthetic urine per gram of gelling agent.

Gel volume in synthetic urine (see Brandt, et al, below) can be determined by forming a suspension of about 0.1-0.2 parts of dried absorbent gelling material to be tested with about 20 parts of synthetic urine. This suspension is maintained at ambient temperature under gentle stirring for about 1 hour so that swelling equilibrium is attained. The gel volume (grams of synthetic urine per gram of absorbent gelling material) is then calculated from the weight fraction of the gelling agent in the suspension and the ratio of the liquid volume excluded from the formed hydrogel to the total volume of the suspension. The preferred absorbent gelling materials useful in this invention will have a gel volume of from about 20 to 70 grams, more preferably from about 30 to 60 grams, of synthetic urine per gram of absorbent gelling material.

Another feature of the most highly preferred absorbent gelling materials relates to the level of extractable polymer material present in said materials. Extractable polymer levels can be determined by contacting a sample of preferred absorbent gelling material with a synthetic urine solution for the substantial period of time (e.g., at least 16 hours) which is needed to reach extraction equilibrium, by then filtering the formed hydrogel from the supernatant liquid, and finally by then determining the polymer content of the filtrate. The particular procedure used to determine extractable polymer content of the preferred absorbent gelling agent buffers herein is set forth in Brandt, Goldman and Inglin; U.S. Patent 4,654,039; Issues March 31,1987, Reissue 32,649, The absorbent gelling materials which are especially useful in the absorbent articles herein are those which have an equilibrium extractables content in synthetic urine of no more than about 17%, preferably no more than about 10% by weight of the absorbent gelling material.

The absorbent gelling materials herein before described are typically used in the form of discrete particles. Such absorbent gelling materials can be of any desired shape, e.g., spherical or semi-spherical, cubic, rod-like polyhedral, etc. Shapes having a large greatest dimension/smallest dimension ratio, like needles and flakes, are also contemplated for use herein. Agglomerates of absorbent gelling material particles may also be used.

The size of the absorbent gelling material particles may vary over a wide range. For reason of industrial hygiene, average particle sizes smaller than about 30 microns are less desirable. Particles having a smallest dimension larger than about 2mm may also cause a feeling of grittyness in the absorbent article, which is undesirable from a consumer aesthetics standpoint. Furthermore, rate of fluid absorption can be affected by particle size. Larger particles have very much reduced rates of absorption. Preferred for use herein are absorbent gelling material s particles substantially all of which have a particle size of from about 30 microns to about 2mm. "Particle Size" as used herein means the weighted average of the smallest dimension of the individual particles.

Typically, the amount of absorbent gelling material particles used in the absorbent articles, will range from 0 gm⁻² to 150gm⁻², preferably from 30gm⁻² to 110gm⁻², more preferably from 55gm⁻² to 85gm⁻².

### Odour control agents

Additional odour control agent or combinations thereof, known in the art for this purpose may be used herein. These agents can typically be classified according to the type of odour the agent is intended to combat. Odors may be chemically classified as being acidic, basic or neutral.

Alternatively, the odor control agents may be categorised with respect to the mechanism by which the malodor detection is reduced or prevented. For example, odor control agents which chemically react with malodorous compounds or with compounds which produce malodorous degradation products thereby generating compounds lacking odor or having an odor acceptable to consumers may also be utilized herein.

Suitable odor control agents for use herein typically include carbonates (e.g., sodium carbonate), bicarbonates (e.g., sodium bicarbonate), phosphates (e.g., sodium phosphate), sulphates (e.g., zinc and copper sulphates), carboxylic acids such as citric acid, lauric acid, boric acid, adipic acid and maleic acid, activated carbons, clays, zeolites, silicas, absorbent gelling materials (AGM) and starches. Such odor control agents and systems are disclosed in more details hereinafter and for example in EP-A- 348 978, EP-A- 510 619, WO 91/12029, WO 91/11977, WO 91/12030, WO 81/01643 and WO 96/06589.

Alternative odor control agents are ion exchange resins such as those described in US 4 289 513 and US 3340875.

Masking agents such as perfumes may also be used as odor control agents herein.

Typically, the absorbent articles may comprise the additional odour control agent or a mixture thereof at a level of from 0 gm⁻² to 600 gm⁻², preferably from 5 to 500 gm⁻², more preferably from 10 gm⁻² to 350 gm⁻² and most preferably from 20 gm⁻² to 200 gm⁻²

### The disposable articles

Preferred breathable absorbent articles herein are pantiliners, feminine napkins, incontinent pads, diapers, nursing pads, and the like. The spores (and optional absorbing gelling material and/or optional additional odor control agent(s)) may be incorporated into the absorbent article by any of the methods disclosed in the art, for example layered on the core of the absorbent article or mixed within the fibres of the absorbent core.

The spores as described herein and optional additional agents are preferably incorporated between two layers of cellulose tissue. Optionally the system may be bonded between two cellulose tissue layers with, for example, a hot melt adhesive or any suitable bonding system, as described in WO 94/01069.

In one embodiment of the present invention the spores and optional additional agents are incorporated in a layered structure in accordance with the disclosure of WO 94/01069 or WO 95/17868. WO 95/17868 describes a layered structure substantially as described in WO 94/01069 with the exception that WO 95/17868 comprises a much higher quantity of absorbent gelling material in the intermediate layer which is between the fibrous layers (at least 120gm⁻²) that would be incorporated as an optional component in the present invention. The intermediate layer comprises in particular a polyethylene powder as thermoplastic material which is mixed with the spore powder as described herein. The mixture is then heated such that the polyethylene melts and glues the laminate layers together.

Adhesive lines are preferably also placed on the edges of the laminate to ensure that the edges of the laminate stick and any loose spores and optional additional agent present do not fall out of the laminate.

Alternatively, the polyethylene powder may be replaced by a conventional glue for instance those commercially available from ATO Findley under the name H20-31® to glue the laminate layers and/or components together. Advantageously this method step allows to avoid the heating step necessary when using polyethylene powder.

The spores may be distributed homogeneously or non homogeneously over the entire absorbent article or in at least one layer of the topsheet or in at least one layer of the backsheet or in at least one layer of the core or any mixture thereof. The spores may be distributed homogeneously or non homogeneously on the whole surface of the desired layer or layers, or on one or several area of the surface layer/layers to which it is positioned (e.g. central area and/or surrounding area like the edges of a layer of the absorbent article) or mixtures thereof. Preferably the spores are located in the core (also called intermediate layer which is positioned between the topsheet and the backsheet). The presence of the spores in the core is preferred because the core collects and absorbs bodily fluids. Thus the close proximity to the substrate contributes to improved antagonistic activity of the spores according to the present invention. Indeed optimum inhibition of the degradation activity by putrefactive bacteria and proliferation of pathogens is obtained.

In one embodiment of the present invention the spores are positioned such that at least a portion of the fluid discharge comes into contact with said spores before the optional absorbent gelling material (e.g., AGM) and/or optional additional odour control agent if present. In particular, the spores can be located in a separate layer from the optional absorbing gelling material and/or optional additional odor control agent if present. In such an embodiment the spores are located towards the topsheet or in the topsheet itself (preferably the secondary topsheet) and the optional absorbing gelling material and/or optional additional odor control agent are located further away from the topsheet than the spores. In one embodiment of the present invention, the spores are positioned in at least one of the topsheet layers and the optional absorbing gelling material and/or optional additional odor control agent, if present, are positioned in the core.

In another embodiment the spores may be located in various layers, i.e., that the total amount of spores is distributed between several layers, e.g., in the topsheet layer and the core. The benefit of this execution are related with the combined action of spores which inhibit the growth of degradative/pathogen bacteria in the core and on the body surface in contact with the absorbent article.

The spores as described herein may be incorporated as a powder or a granulate. When used in a granulate or particulate form the spores as described herein and the optional absorbing gelling material and/or optional odor control agent may be granulated separately and then mixed together or granulated together.

Suitable breathable absorbent article according to the present invention include those described as follows:

### Backsheet

According to the present invention, the absorbent articles comprise as an essential component a breathable backsheet. The primary role of the breathable backsheet is to prevent the extrudes absorbed and contained in the absorbent article from wetting articles that contact the absorbent article such as pyjamas and undergarments. In order to achieve this the backsheet typically extends across the whole of the absorbent structure and may extend into and form part of or all of sideflaps, side wrapping elements or wings. In addition to the prevention of liquid transport through the backsheet however, the breathable backsheet also permits the transfer of water vapor and preferably both water vapor and air through it and thus allows the circulation of air into and out of the backsheet and the absorbent article itself.

Suitable breathable backsheets for use herein include all breathable backsheets known in the art. In principle there are two types of breathable backsheets, single layer breathable backsheets which are breathable and impervious to liquids and backsheets having at least two layers, which in combination provide both breathability and liquid imperviousness.

Suitable single layer breathable backsheets for use herein include those described for example in GB A 2184 389, GB A 2184 390, GB A 2184 391, US 4 591 523, US 3 989 867 US 3 156 242 and European Patent Application number 95120653.1.

Suitable dual or multi layer breathable backsheets for use herein include those exemplified in US 3 881 489, US 4 341 216, US 4 713 068, US 4 818 600, EPO 203 821, EPO 710 471, EPO 710 472, European Patent Application numbers 95120647.3, 95120652.3, 95120653.1 and 96830097.0.

Particularly preferred are backsheets meeting the requirements as defined in European Patent Application number 96830343.8 and more preferably wherein the absorbent article also meets the requirements as described therein.

According to the present invention the breathable backsheet comprises at least one, preferably at least two water vapor permeable layers. Suitable water vapor permeable layers include 2 dimensional, planar micro and macro-porous films, monolithic films, macroscopically expanded films and formed apertured films. According to the present invention the apertures in said layer may be of any configuration, but are preferably spherical or oblong. The apertures may also be of varying dimensions. In a preferred embodiment the apertures are preferably evenly distributed across the entire surface of the layer, however layers having only certain regions of the surface having apertures is also envisioned.

2 dimensional planar films as used herein have apertures having an average diameter of from 5 micrometers to 200 micrometers. Typically, 2-dimensional planar micro porous films suitable for use herein have apertures having average diameters of from 150 micrometers to 5 micrometers, preferably from 120 micrometers to 10 micrometers, most preferably from 90 micrometers to 15 micrometers. Typical 2 dimensional planar macroporous films have apertures having average diameters of from 200 micrometers to 90 micrometers. Macroscopically expanded films and formed apertured films suitable for use herein typically have apertures having diameters from 100 micrometers to 500 micrometers. Embodiments according to the present invention wherein the backsheet comprises a macroscopically expanded film or an apertured formed film, the backsheet will typically have an open area of more than 5%, preferably from 10% to 35% of the total backsheet surface area.

Suitable 2 dimensional planar layers of the backsheet may be made of any material known in the art, but are preferably manufactured from commonly available polymeric materials. Suitable materials are for example GORE-TEX (TM) or Sympatex (TM) type materials well known in the art for their application in so-called breathable clothing. Other suitable materials include XMP-1001 of Minnesota Mining and Manufacturing Company, St. Paul, Minnesota, USA. As used herein the term 2 dimensional planar layer refers to layers having a depth of less than 1mm, preferably less than 0.5mm, wherein the apertures have an average uniform diameter along their length and which do not protrude out of the plane of the layer. The apertured materials for use as a backsheet in the present invention may be produced using any of the methods known in the art such as described in EPO 293 482 and the references therein. In addition, the dimensions of the apertures produced by this method may be increased by applying a force across the plane of the backsheet layer (i.e. stretching the layer).

Suitable apertured formed films include films which have discrete apertures which extend beyond the horizontal plane of the garment facing surface of the layer towards the core thereby forming protuberances. The protuberances have an orifice located at their terminating ends. Preferably said protuberances are of a funnel shape, similar to those described in US 3, 929,135. The apertures located within the plane and the orifices located at the terminating end of protuberance themselves maybe circular or non circular, provided the cross sectional dimension or area of the orifice at the termination of the protuberance is smaller than the cross sectional dimension or area of the aperture located within the garment facing surface of the layer. Preferably said apertured preformed films are uni directional such that they have at least substantially, if not complete one directional fluid transport towards the core. Suitable macroscopically expanded films for use herein include films as described in for example in US 637 819 and US 4 591 523.

Suitable macroscopically expanded films for use herein include films as described in for example US 4 637 819 and US 4 591 523.

Suitable monolithic films include Hytrel™, available from DuPont Corporation, USA, and other such materials as described in Index 93 Congress, Session 7A "Adding value to Nonwovens", J-C. Cardinal and Y. Trouilhet, DuPont de Nemours International S.A., Switzerland.

According to the present invention the backsheet may comprise in addition to said water vapor permeable layer additional backsheet layers. Said additional layers may be located on either side of said water vapor permeable layer of the backsheet. The additional layers may be of any material, such as fibrous layers or additional water vapor permeable layers as described herein above.

In a particularly preferred embodiment herein a dual or multiple layer breathable backsheet composite is used in the absorbent article. According to the present invention suitable breathable backsheets for use herein comprise at least a first and a second layer. The first layer is positioned between the garment facing surface of the absorbent core and the wearer facing surface of the second layer. It is oriented such that it retards or prevents liquid from passing from the absorbent core towards the outside while allowing free air flow and water vapor through it. The second layer provides water vapor and air permeability so as to support breathability of the article. In addition to water vapor permeability the air permeability is desirable in order to further improve the comfort benefit from the breathability of the article.

Such a first layer is preferably in direct contact with the absorbent core. It provides air and water vapor permeability by being apertured. Preferably this layer is made in accordance with the aforementioned US-A-5,591,510 or PCT WO-97/03818, WO-97/03795. In particular, this layer comprises a polymeric film having capillaries . The capillaries extend away from the wearer facing surface of film at an angle which is less than 90 degrees. Preferably the capillaries are evenly distributed across the entire surface of the layer, and are all identical. However, layers having only certain regions of the surface provided with apertures, for example only an area outside the region aligned with the central loading zone of the absorbent core, maybe provided with such capillaries.

Methods for making such three-dimensional polymeric films with capillary apertures are identical or similar to those found in the apertured film topsheet references, the apertured formed film references and the micro-/macroscopically expended film references cited above. Typically a polymeric film such as a polyethylene (LDPE, LLDPE, MDPE, HDPE or laminates thereof) or preferably a monolithic polymeric film is heated close to its melting point and exposed through a forming screen to a suction force which pulls those areas exposed to the force into the forming apertures which are shaped such that the film is formed into that shape and, when the suction force is high enough, the film breaks at its end thereby forming an aperture through the film.

Especially using a monolithic polymer film as the material for the first layer provides water vapor permeability even under stress conditions. While the apertures provide air permeability during "leakage safe" situations but close the capillaries under stress conditions the monolithic material maintains water vapor permeability in such a case. Preferred breathable monolithic film materials for use herein are those having a high vapor exchange. Suitable monolithic films include Hytrel (TM), available from DuPont Corporation, USA, and other such materials as described in Index 93 Congress, Session 7A "Adding value to Nonwovens", J-C. Cardinal and Y. Trouilhet, DuPont de Nemours international S.A, Switzerland.

Various forms, shapes, sizes and configurations of the capillaries are disclosed in EP-A-934735 and EP-A-934736 both of which are herein incorporated for reference. In particular the apertures form capillaries which have side walls . The capillaries extend away from the wearer facing surface of the film for a length which typically should be at least in the order of magnitude of the largest diameter of the aperture while this distance can reach up to several times the largest aperture diameter. The capillaries have a first opening in the plane of the garment facing surface of the film and a second opening which is the opening formed when the suction force (such as a vacuum) in the above mentioned process creates the aperture. Naturally the edge of the second opening may be rugged or uneven, comprising loose elements extending from the edge of the opening. However, it is preferred that the opening be as smooth as possible so as not to create a liquid transport entanglement between the extending elements at the end of the second opening of the capillary with the absorbent core in the absorbent article (in contrast this may be desirable for apertured film topsheets where such loose elements provide the function of sucker feet). The capillaries in the first layer of the breathable backsheet allow air and water vapor permeability which is not hindered by them being slanted at an angle or by the shape. At the same time the slanting and shaping will allow the capillaries to close under pressure excerpted from the wearer facing side on them such that liquid transport through the capillaries towards the outside of the article becomes nearly impossible. Hence these three-dimensional formed film layers are highly preferable in the context of breathable absorbent articles and in particular so with the additional second outer layer which is provided as hereinafter explained.

The second outer layer of the breathable backsheet according to the present invention is a fibrous nonwoven web having a basis weight of less than 40 g/m2, preferably of less than 28 g/m2. More preferably, the second outer layer is a fibrous nonwoven web formed by a layered composite of a meltblown nonwoven layer made from synthetic fibers having a basis weight of less than 13 g/m2 and of a spunbonded nonwoven layer also made from synthetic fibers.

In the most preferred embodiment herein the backsheet comprises at least a first layer of a resilient, three dimensional web which consists of a liquid impervious polymeric film having apertures forming capillaries which are not perpendicular to the plane of the film but are disposed at an angle of less than 90° relative to the plane of the film, and at least a second breathable layer of a porous web which is a fibrous nonwoven composite web of a meltblown nonwoven layer made from synthetic fibers having a basis weight of less than 13 g/m2 and of a spunbonded nonwoven layer made from synthetic fibers.

Using as the breathable backsheet in the absorbent article of the present invention, a backsheet comprising at least one breathable layer of a resilient, three dimensional web which consists of a liquid impervious polymeric film having apertures forming capillaries which are not perpendicular to the plane of the film but are disposed at an angle of less than 90° relative to the plane of the film, and at least another breathable layer of a porous web which consists of a fibrous nonwoven web having a basis weight of less than 40 g/m2 (particularly of about 28 g/m2), further contributes to the outstanding benefit of the present invention. Indeed these backsheet function very well in term of comfort, soiling of the user panty, dryness, etc. while providing additional comfort due to the reduced basis weight of the non-woven layer. This reduction of basis weight also provides an improved material consumption structure of the whole article.

### Absorbent core

According to the present invention the absorbent articles may further comprise a topsheet and absorbent core. The absorbent material or core can be a fluffy fibrous absorbent core, comprising hydrogel particles if desired, or laminated tissues with or without particulate materials including hydrogel particles. The absorbent core fibres can be any of those known in the art including cellulose fibres or polymeric fibres rendered absorbent or even non absorbent matrix fibres. Also tissues of sufficient basis weight and absorbency can be used in the absorbent core according to the present invention.

### Topsheet

According to the present invention the topsheet may comprise a single layer or a multiplicity of layers. In a preferred embodiment the topsheet comprises a first layer which provides the user facing surface of the topsheet and a second layer between the first layer and the absorbent structure/core. The topsheet provides a layer through which the liquids to be absorbed penetrate to the absorbent material.

The topsheet as a whole and hence each layer individually needs to be compliant, soft feeling, and non-irritating to the wearer's skin. It also can have elastic characteristics allowing it to be stretched in one or two directions. Typically, the topsheet extends across the whole of the absorbent structure and can extend into and form part of or all of the preferred sideflaps, side wrapping elements or wings. According to the present invention the topsheet may be formed from any of the materials available for this purpose and known in the art, such as woven non woven materials, polymeric materials such as apertured formed thermoplastic films, apertured plastic films and hydroformed thermoplastic films, thermoplastic scrims or combinations thereof. Suitable woven and non woven materials can be comprised of natural fibers (e.g., wood or cotton fibers), synthetic fibers (e.g., polymeric fibers such as polyester, polypropylene or polyethylene fibers) or from a combination of natural and synthetic fibers or bi-/multi- component fibers and are preferably hydrophobic.

In a preferred embodiment of the present invention at least one of the layers of the topsheet comprises a hydrophobic, liquid permeable apertured polymeric film. Preferably, the upper layer is provided by a film material having apertures which are provided to facilitate liquid transport from the wearer facing surface towards the absorbent structure, as detailed for example in US 3 929 135, US 4 151 240, US 4 319 868, US 4 324 426, US 4 343 314, US 4 463 045, US 5 006 394 and US 4 591 523. Apertured formed films are especially preferred for the topsheets because they are pervious to body exudates and yet non absorbent and have a reduced tendency to allow fluids to pass back through and rewet the wearer's skin. Thus, the surface of the formed film that is in contact with the body remains dry, thereby reducing body soiling and creating a more comfortable feel for the wearer. Particularly preferred micro apertured formed film topsheets are disclosed in US 4 609 518 and US 4 629 643.

Topsheets having not a homogeneous distribution of liquid passage ways but only a portion of the topsheet comprising liquid passage ways are also contemplated by the present invention. Typically such topsheets would have the liquid passage ways oriented such that they result in a centrally permeable and peripherally impermeable topsheet for liquids.

The wearer facing surface of the formed film topsheet can be hydrophilic so as to help liquid to transfer though the topsheet faster than if the body surface was not hydrophilic. In a preferred embodiment, surfactant is incorporated into the polymeric materials of the formed film topsheet such as is described in WO 93/09741. Alternatively, the wearer facing surface of the topsheet can be made hydrophilic by treating it with a surfactant such as is described in US 4 950 254.

According to the present invention the absorbent article is constructed by joining the various elements such as topsheet, backsheet and absorbent core by any means well known in the art. For example the backsheet and/ or topsheet may be joined to the absorbent core or to each other by a uniform continuous layer of adhesive, a patterned layer of adhesive, or an array of separate lines, spirals or spots of adhesive. Alternatively, the elements may be joined by heat bonds, pressure bonds, ultra sonic bonds, dynamic mechanical bonds or any other suitable joining means known in the art and any combination thereof. Preferably the breathable backsheet is bonded to other elements of the absorbent article so as to minimize and preferably eliminate any reduction in the vapor permeability of the backsheet .

According to the present invention the absorbent article may find utility as sanitary napkins, panty liners, adult incontinence products and baby diapers. The present invention finds particular susceptibility as sanitary napkins and panty liners. Thus in addition to the components described herein above, the absorbent article may also comprise all those features and parts which are typical for products in the context of their intended use such as wings and side flaps, undergarment adhesive means, release paper, wrapping elements, fastening means and the like.

### The present invention is further illustrated by the following examples

### Examples

### Example 1:

This is an example of a pantyliner according to the present invention and is a modified panty liner based on Always "Alldays Duo Active" manufactured by Procter & Gamble, Germany. The topsheet is a film/non woven composite {film supplier code BPC 5105 CPM BP Chemical Germany, non woven supplier code ARBO TB/BI Mequinenza Spain}. The core material is a tissue laminate (13.2 cm x 4.0 cm) composed of a 2 layers of airlayed tissue of 55 g/m² basis weight {available from Unikay Italy under the supplier code Unikay 303 LF}. Between the two tissue layers the laminate contains *L. sporogenes* spores Lactospore® commercially available from Sabinsa Corporation (Sochim International S.P.A. Milano) at a basis weight of 55 g/m². This corresponds to about 10^{*}10⁹ spores (ten billions of spores per pantiliner). 10^{*}10⁹ spores correspond to 10^{*}10⁹ cfu of *L.sporogenes* per pantiliner.

The backsheet comprises two layers a first layer and a second layer. The first layer is in contact with the absorbent tissue and the second layer. The second layer is in contact with the first layer and the undergarment of the wearer. The first layer is a formed apertured film (CPT) made of Low Density PE {supplied by Tredegar Film Products B.V. Holland under the manufacturing code X-1522}. The second layer is composed of a nonwoven laminate {13MB/16SB manufactured by Corovin GmbH in Germany under the trade name MD 2005}. The nonwoven laminate is composed of 16 g/m² spunbond and 13 g/m² meltblown. Each backsheet layer is joined over the full surface by a extensively overlapped spiral glue application at a basis weight of approximately 8 g/m². The glue utilised for attachment of both backsheet layers was supplied by SAVARE' SpA. Italy (under the material code PM17).

### Example 2:

Example 2 is identical to example 1 except that the second layer of the backsheet has been replaced by a nonwoven laminate composed of 16g/m² spunbond and 6 g/m² meltblown {supplied under the code of SM 22-6PH by Union SpA, Italy}.

### Example 3:

Example 3 is identical to example 1 except that AGM was added (available from DOW Chemicals Germany under the supplier code; DOW XZ 95890.1) at a basis weight of 60 g/m².

### Example 4:

Example 4 is identical to example 3 except that the second layer of the backsheet has been replaced by a nonwoven laminate composed of 16g/m² spunbond and 6 g/m² meltblown {supplied under the code of SM 22-6PH by Union SpA, Italy}.

### Example 5:

This is an example of a sanitary napkin according to the present invention. The sanitary napkin is based on an Always Ultra sanitary napkin available from Procter & Gamble Germany which has been modified. The topsheet is a CPM material available from Tredegar Film Products B. V. Holland under the code X-1522. The core material is a tissue laminate (20.7 cm x 7.0 cm) composed of a 2 layers of airlayed tissue of 55 g/m² basis weight {available from Unikay Italy under the supplier code Unikay 303 LF}. Between the two tissue layers the laminate contains an odor control system of AGM (available from DOW Chemicals Germany under the supplier code; DOW XZ 95890.1) at a basis weight of 60 g/m², a zeolite (available from Degussa Germany under the supplier code; Wessalith CS) at a basis weight of 61 g/m² and *L. sporogenes* spores Lactospore® commercially available from Sabinsa Corporation (Sochim International S.P.A. Milano) at a basis weight of 55 g/m². This corresponds to about 10^{*}10⁹ spores (ten billions of spores per napkin). 10^{*}10⁹ spores correspond to 10*10⁹ cfu of *L.sporogenes* per napkin.

The core laminate was manufactured and supplied by Korma Italy (under the experimental manufacturing code: XA 070.01.003). The sanitary napkin has a multi-layer breathable backsheet comprising a formed apertured film backsheet layer and a second nonwoven layer. The first layer is a blend of low and high density PE with a crush resistant hexagonal hole configuration {supplied by Tredegar Film Products B.V. Holland under the manufacturing code AS 225 HD 25}. The second layer is an improved nonwoven laminate composed of 3 layers with basis weights 14g/m² spunbond - 20 g/m² meltblown - 14 g/m² spunbond (manufactured by Corovin GmbH in Germany under the trade name MD 3005).

### Example 6:

Example 6 is identical to example 5 except that the second layer of backsheet has been replaced by a microporous layer (manufactured by Exxon Chemical Company in Illinois under the name Exxon XBF 112W) composed of Low Density PE and calcium carbonate particles at basis weight of 35 g/m2.

### Example 7 :

Example 7 is identical to example 5 except that the first layer of backsheet has been replaced by an improved resilient tri-dimensional web (supplied by Tredegar Film Products B.V. Holland under the manufacturing code V174LD40) which consist of a blend of low density PE having apertures forming capillaries which are not perpendicular to the plane of the film but are disposed at an angle of less than 90° relative to the plane of the film and that the second layer of the backsheet has been replaced by a nonwoven laminate manufactured by Corovin GmbH (BBA Group) in Germany under the manufacturing code V 8/6. The nonwoven laminate is composed of 16 g/m² basis weight spunbond fiber layer and 11.5 g/m² basis weight meltblown fiber layer (thus a total basis weight of 27.5 g/m2).

## Claims

1. An absorbent article comprising a liquid permeable topsheet, a breathable backsheet and an absorbent core, said core being intermediate said topsheet and said backsheet, said absorbent article comprising spores which have the ability to germinate into microorganisms which exhibit antagonistic properties against undesirable strains of microorganisms.

2. An absorbent article according to claim 1, wherein said spores are spores of the species *Lactobacillus sporogenes* and/or of the genus *Sporolactobacillus.*

3. An article according to any of the preceding claims wherein the absorbent article comprises more than 10² cfu, preferably more than 10⁴ cfu, more preferably from 10⁵ cfu to 10¹² cfu and most preferably from 10⁶ to 10¹⁰ cfu of said antagonistic microorganisms.

4. An article according to any of the preceding claims, which further comprises an absorbing gelling material or a mixture thereof.

5. An article according to claim 4, wherein the level of the absorbing gelling material or a mixture thereof in the article is from 0 gm⁻² to 150gm⁻², preferably from 30gm⁻² to 110gm⁻², more preferably from 55gm⁻² to 85gm⁻².

6. An absorbent article according to any one of the preceding claims, which further comprises at least one additional odor control agent.

7. An absorbent article according to claim 6, wherein said additional odor control agent is selected from the group consisting of silicas, zeolites, carbons, starches, cyclodextrine, kieselguhr, clays, ion exchange resins, carbonates, bicarbonates, phosphates, sulphates, masking agents, and mixtures thereof and preferably is silicate, zeolite or a combination thereof.

8. An article according to any of the preceding claims 6 to 7 which comprises from 0 gm⁻² to 600 gm⁻², preferably from 5 to 500 gm⁻², more preferably from 10 gm⁻² to 350 gm⁻² of said additional odor control agent or a mixture thereof.

9. An absorbent article according to any one of the preceding claims, wherein said breathable backsheet comprises at least one layer selected from an apertured polymeric film or a 2-dimensional planar apertured film.

10. An absorbent article according to claim 9, wherein said layer is a 2 dimensional planar apertured layer, wherein said apertures have an average diameter of from 150 micrometers to 5 micrometers.

11. An absorbent article according to claim 9, wherein said layer is an apertured polymeric film, wherein said apertures have an average diameter of from 100 micrometers to 500 micrometers.

12. An absorbent article according to claim 9, wherein said breathable backsheet comprises at least two layers, a first layer comprising an apertured layer and a second layer comprising a fibrous layer.

13. An absorbent article according to claim 12, wherein said breathable backsheet comprises at least a first layer of a resilient, three dimensional web which consists of a liquid impervious polymeric film having apertures forming capillaries which are not perpendicular to the plane of the film but are disposed at an angle of less than 90° relative to the plane of the film, and at least a second breathable layer being a fibrous nonwoven web made from synthetic fibers having a basis weight of less than 40 g/m2.

14. An absorbent article according to any one of the preceding claims, wherein said article is a sanitary napkin, a nursing pad, baby diaper or a panty liner.

15. The use of spores which have the ability to germinate into microorganisms which exhibit antagonistic properties against undesirable strains of microorganisms, in a breathable absorbent article, comprising a liquid permeable topsheet, an absorbent core and a breathable backsheet, for reducing leakage/wet through.

16. The use of spores which have the ability to germinate into microorganisms which exhibit antagonistic properties against undesirable strains of microorganisms, in an absorbent article, preferably a breathable absorbent article, comprising a wearer facing surface and a garment facing surface for improved dryness of the wearer facing surface.

17. The use according to claim 16 wherein said absorbent article further comprises a topsheet, a backsheet and an absorbent core, said core being located in between said topsheet and said backsheet and wherein said topsheet provides said wearer facing surface.
